# EUROPEAN PATENT SPECIFICATION

(11) **EP 0 348 527 B1**
(45) Date of publication and mention of the grant of the patent: **23.06.1993**
(21) Application number: 89900879.1
(22) Date of filing: 23.12.1988
(51) Int. Cl.: A61K 31/505, A61K 9/06, C07D 471/04

(54) **ANTIALLERGIC EYE DROP**
ANTIALLERGISCHE AUGENTROPFEN
COLLYRE ANTIALLERGIQUE

(30) Priority: 25.12.1987 JP 331025/87
(43) Date of publication of application: 03.01.1990
(73) Proprietor: SANTEN PHARMACEUTICAL CO., LTD., Higashiyodogawa-ku Osaka-shi Osaka 533 (JP)
(72) Inventor: MORITA, Takakazu 302 Kitasakurazuka Park Heim, Toyonaka-shi Osaka 560 (JP); ISO, Tadashi, Kawachinagano-shi Osaka 586 (JP); KAWASHIMA, Youichi, Kyoto-shi Kyoto 610-11 (JP); HIKIDA, Mitsushi, Takatsuki-shi Osaka 569 (JP)
(74) Representative: Pearce, Anthony Richmond
(86) International application number: PCT/JP88/01296
(87) International publication number: WO 89/06130

(56) References cited:
- JP-A- 5 436 294
- PATENT ABSTRACTS OF JAPAN, vol. 7, no. 14 (C-146)[1159], 20th January 1983; & JP-A-57 171 914 (TOUKAI DAIGAKU) 22-10-1982
- THE JAPAN JOURNAL OF PHARMACOLOGY, vol. 48, no. 1, September 1988, pages 91-101; Y. YANAGIHARA et al.: "Immunopharmacological studies on TBX, a new antiallergic drug (1) inhibitory effects on passive cutaneous anaphylaxis in rats and guinea pigs"

## Description

This invention offers opthalmic solutions which are used for treatment of allergic eye diseases such as allergic conjunctivitis.

Japanese Patent Publication ( Publication No. Sho 60-50197 ) discloses that the compound of the formula [I] and salts thereof ( hereinafter called as Compound [I]) are excellent anti-allergic drugs. 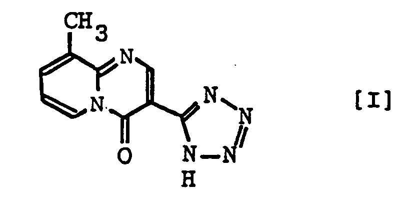
(ie 9-methyl-3-(1H-tetrazol-5-yl)-4H-pyrido[1,2-a]pyrimidin-4-one)
But, ophthalmic application of Compound [I] has not been studied, so, it is necessary to examine preparation methods of ophthalmic formulations and study the effect on allergic eye diseases.

As the result of our precise studies on preparation methods of ophthalmic formulations and the effect on allergic eye diseases, we found that Compound [I] has an excellent anti-allergic effect in eye and has a possibility to be applied to stable and low irritative ophthalmic solutions.

This invention relates to an aqueous anti-allergic ophthalmic solution comprising an aqueous anti-allergic ophthalmic solution containing 9-methyl-3-(1H-tetrazol-5-yl)-4H-pyrido[1,2-a]pyrimidin-4-one potassium salt in a concentration of from 0.01 to 1%, and a buffer selected from a sodium phosphate buffer, a boric acid buffer and a sodium borate buffer.

Compound [I] is already known by US-A-4122274 to have an excellent anti-allergic effect. However, the application to ophthalmic solutions and its effect on allergic eye diseases have not been known.

Topical diseases such as eye diseases are efficiently treated by topical application of a drug and it is necessary to study a topical application of oral drug to ophthalmics.

Hereinafter, 9-methyl-3-(1H-tetrazol-5-yl)-4H-pyrido[1,2-a]pyrimidin-4-one potassium salt will be referred to as Compound A. It is an excellent anti-allergic drug.

Important factors for ophthalmic solutions are not only to have excellent efficacy but also low irritation because ophthalmic compositions are administered directly to the eye which is a highly sensitive organ.

Particularly, patients with allergic diseases complain of a strong pain in the eye, so less irritative ophthalmic composition are desired to treat such patients.

As the result of the experiment using eye drops of the ophthalmic solution of the present invention, we found that the eye drops cause little eye irritation and the ophthalmic solution of this invention is well applicable to anti-allergic ophthalmics.

Furthermore, in case of eye drops, stability in solution is required, as opposed to oral drugs, because the ingredients should be dissolved. We examined the stability of the eye drops of compound A. As the result of a preservation test lasting 6 months at 40°C and 75% relative humidity, no notable changes were observed and the eye drops showed good stability.

These properties cannot be presumed from the properties of oral preparations and can be found only from the application study to ophthalmics.

We examined the efficacy of the ophthalmic solution, which is the most important property, and found that Compound A showed a strong anti-allergic effect by topical application, the details of which are described later under the heading of pharmacological test using eye drops of Compound A, and that the ophthalmic solution of this invention was very useful for treatment of allergic eye diseases such as allergic conjunctivitis.

Furthermore, to apply a medical substance to eye drops, the solubility of the substance is an important factor.

If a substance is easily soluble in water, no particular consideration is necessary. But, if a medical substance is hardly soluble in water, or if a substance, once dissolved in water, has a tendency to crystallize during storage, many kinds of studies are required to formulate such a substance into eye drops.

Compound A, which is an excellent anti-allergic drug, is a water soluble substance, but it has a tendency to crystallize after having been dissolved in water. So, such problem about solubility has to be solved to formulate Compound A in eye drops.

A method using a solubilizer such as polyvinyl alcohol is considered first. However, the problem mentioned above can not be solved using such method and another idea is required. Furthermore, in case of eye drops, when the concentration of potassium is increased, there is a risk of causing corneal damage. So, particular consideration is required to apply such a substance to ophthalmics.

When the dosage frequency is low, potassium concentration is not an important problem. But, when continuous administration of the ophthalmics solution is required to treat eye diseases, it is important to lower the potassium concentration.

As the result of our intensive studies to solve such problems, we found that the problems could be solved immediately by using a sodium phosphate buffer, a boric acid buffer or a sodium borate buffer. We found that especially superior eye drops could be prepared by using a combination of disodium hydrogen phosphate and sodium dihydrogen phosphate, disodium hydrogen phosphate and potassium dihydrogen phosphate, boric acid and sodium borate or boric acid and monoethanolamine.

The combination amount and ratio of the buffer depend on the concentration of Compound A. However, for the specified concentration range for Compound A of 0.01 - 1%, the following are preferable combinations:-
Disodium hydrogen phosphate and sodium dihydrogen phosphate are 0.1 - 2% and 0.002 - 1% respectively. Disodium hydrogen phosphate and potassium dihydrogen phosphate are 0.1 - 1% and 0.002 - 0.7% respectively. Boric acid and sodium borate are 0.3 - 2% and 0.3 - 1% respectively. Boric acid and monoethanolamine are 0.3 - 2% and 0.1 - 1% respectively.

Examples of additives usually combined in ophthalmic solutions are tonicity agents such as sodium chloride, potassium chloride and concentrated glycerin, stabilizers such as sodium sulfite and disodium edetate, preservatives such as benzalkonium chloride, surfactants such as polysorbate 80 and polyoxyethylene hydrogenated castor oil, pH adjusting agents such as sodium hydroxide, potassium hydroxide and hydrochloric acid and eye ointment bases such as vaseline and liquid paraffin.

The pH value of the ophthalmic solution of this invention can be adjusted according to the range acceptable in ophthalmics, but in eye drops of Compound A, the pH is preferably 7 - 9.

The typical preparation method of the ophthalmic solution of this invention is that Compound A is added to sterile purified water to which has been added tonicity agent, buffering agent, stabilizer, preservatives, surfactant, pH adjusting agent, etc.

### BEST MODE TO MAKE THE INVENTION

### Example 1

| Formulation A | |
|---|---|
| Compound A | 0.3g |
| sodium dihydrogen phosphate | 0.01g |
| disodium hydrogen phosphate | 0.35g |
| concentrated glycerine | 2.0g |
| benzalkonium chloride | 0.005g |
| sterile purified water | q.s. |
| total | 100ml |

### Preparation method :

Sodium dihydrogen phosphate, disodium hydrogen phosphate, concentrated glycerin and benzalkonium chloride were dissolved in 80 ml of sterile purified water and then Compound A was added to the solution. After dissolving Compound A, sterile purified water was added to adjust the total volume to 100ml.

Eye drops of the formulations B - E were prepared by the similar method as Example 1.

| Formulation B | |
|---|---|
| Compound A | 0.05g |
| sodium dihydrogen phosphate | 0.04g |
| disodium hydrogen phosphate | 1.4g |
| potassium chloride | 0.7g |
| benzalkonium chloride | 0.005g |
| sterile purified water | q.s. |
| total | 100ml |

| Formulation C | |
|---|---|
| Compound A | 1.0g |
| boric acid | 1.8g |
| monoethanolamine | 0.6g |
| sodium sulfite | 0.2g |
| benzalkonium chloride | 0.005g |
| sterile purified water | q.s. |
| total | 100ml |

| Formulation D | |
|---|---|
| Compound A | 0.01g |
| boric acid | 0.81g |
| sodium borate | 0.67g |
| potassium chloride | 0.24g |
| benzalkonium chloride | 0.005g |
| sterile purified water | q.s. |
| total | 100ml |

| Formulation E | |
|---|---|
| Compound A | 0.1g |
| sodium dihydrogen phosphate | 0.008g |
| disodium hydrogen phosphate | 0.32g |
| concentrated glycerin | 1.8g |
| benzalkonium chloride | 0.005g |
| sterile purified water | q.s. |
| total | 100ml |

### Example 2

| Formulation F | |
|---|---|
| Compound A | 0.5g |
| boric acid | 0.7g |
| sodium chloride | 0.13g |
| benzalkonium chloride | 0.005g |
| potassium hydroxide | q.s. |
| sterile purified water | q.s. |
| total | 100ml |

### Preparation method :

Boric acid, sodium chloride and benzalkonium chloride were dissolved in 80 ml of sterile purified water. Compound A was added to the solution, pH was adjusted to 8.5 with potassium hydroxide and sterile purified water was added to the solution to adjust the total volume to 100ml.

Eye drops of the formulation G - I were prepared by the similar method as Example 2.

| Formulation G | |
|---|---|
| Compound A | 0.01g |
| sodium dihydrogen phosphate | 0.73g |
| disodium hydrogen phosphate | 0.71g |
| benzalkonium chloride | 0.005g |
| potassium chloride | 0.21g |
| potassium hydroxide | q.s. |
| sterile purified water | q.s. |
| total | 100ml |

| Formulation H | |
|---|---|
| Compound A | 0.01g |
| potassium dihydrogen phosphate | 0.6g |
| disodium hydrogen phosphate | 0.3g |
| benzalkonium chloride | 0.01g |
| potassium chloride | 0.5g |
| sodium hydroxide | q.s. |
| sterile purified water | q.s. |
| total | 100ml |

| Formulation I | |
|---|---|
| Compound A | 1.0g |
| sodium dihydrogen phosphate | 0.3g |
| disodium hydrogen phosphate | 0.15g |
| concentrated glycerin | 1.4g |
| benzalkonium chloride | 0.005g |
| potassium hydroxide | q.s. |
| sterile purified water | q.s. |
| total | 100ml |

### PHARMACOLOGICAL TEST

The inhibition effect on the anaphylactic reaction is usually measured as an indication of efficacy against allergic diseases.

We examined the inhibition effect on the anaphylactic reaction by the ophthalmic solution of this invention, using a rat conjunctiva model in which a passive cutaneous anaphylactic reaction was induced. Sodium cromoglycate, which has been applied to anti-allergic ophthalmics, was used as a comparative drug.

### ( experimental method )

According to the method of ISO et. al. ( Ophthalmic Res., 12, 9 (1980) ), we examined the inhibition effects of test compounds on allergy of rat conjunctiva, using four times diluted antiserum ( PCA titer 1:32 ) prepared by Mota's method ( Life Sci., 12, 917 (1963) ). Ten µl of eye drops, which had been prepared by dissolving the test compound in saline and adjusted pH 7.5, were applied 5 and 15 minutes before challenge of antigen.

### ( result )

| test compound | concentration(%) | inhibition effect of anaphylactic reaction(%) |
|---|---|---|
| Compound A | 0.01 | 55.5 |
| Compound A | 0.1 | 88.5 |
| Compound A | 1.0 | 94.8 |
| Sodium cromoglycate | 1.0 | 9.5 |

The percent inhibition of the anaphylactic reaction by the eye drops of this invention is over 50% even when the concentration of Compound A is as low as 0.01%. In case of eye drops containing 1.0% of Compound A, the percent inhibition was over 90%.

The anti-allergic effect of Compound A by topical application is superior to that of sodium cromoglycate. The results prove the utility of the ophthalmic solution of this invention.

### IRRITATION TEST

Measurement of blinking rate and Draze test using rabbit are usually applied as an indicator of eye irritation caused by ophthalmic solutions.

We examined the irritation, comparing the ophthalmic solution of this invention with the vehicle of it.

As one of the examples, the result using the ophthalmic solution of the Formulation I in Example 2 is shown below. Each blinking rate one minute after one drop application of the eye drops of the Formulation I or one drop of its vehicle is low such as 0.8 times ( mean value of 5 rabbits ), and irritation by medicament was not recognized.

After 10 times applications of the eye drops of the Formulation I or its vehicle, we scored according to the improved Draze method ( Fukui et. al., Gendai no Rinsyo 4, 277 (1970) ) and found no damage in either case. The results showed that the irritation of the ophthalmic solution of this invention was weak.

## Claims

1. An aqueous anti-allergic ophthalmic solution containing 9-methyl-3-(1H-tetrazol-5-yl)-4H-pyrido[1,2-a]pyrimidin-4-one potassium salt in a concentration of from 0.01 to 1%, and a buffer selected from a sodium phosphate buffer, a boric acid buffer and a sodium borate buffer.

2. An aqueous anti-allergic ophthalmic solution as claimed in claim 1, wherein the buffer comprises disodium hydrogen phosphate and sodium dihydrogen phosphate.

3. An aqueous anti-allergic ophthalmic solution as claimed in claim 2, wherein the concentrations of the disodium hydrogen phosphate and the sodium dihydrogen phosphate are 0.1 to 2% and 0.002 to 1%, respectively.

4. An aqueous anti-allergic ophthalmic solution as claimed in claim 1, wherein the buffer comprises disodium hydrogen phosphate and potassium dihydrogen phosphate.

5. An aqueous anti-allergic ophthalmic solution as claimed in claim 4, wherein the concentrations of the disodium hydrogen phosphate and the potassium dihydrogen phosphate are 0.1 to 1% and 0.002 to 0.7%, respectively.

6. An aqueous anti-allergic ophthalmic solution as claimed in claim 1, wherein the buffer comprises boric acid and sodium borate.

7. An aqueous anti-allergic ophthalmic solution as claimed in claim 6, wherein the concentrations of the boric acid and the sodium borate are 0.3 to 2% and 0.3 to 1%, respectively.

8. An aqueous anti-allergic ophthalmic solution as claimed in claim 1, wherein the buffer comprises boric acid and monoethanolamine.

9. An aqueous anti-allergic ophthalmic solution as claimed in claim 8, wherein the concentrations of the boric acid and the monoethanolamine are 0.3 to 2% and 0.1 to 1%, respectively.

10. A process for preparing an aqueous anti-allergic ophthalmic solution, comprising dissolving 9-methyl-3-(1H-tetrazol-5-yl)-4H-pyrido[1,2-a]pyrimidin-4-one potassium salt in a concentration of from 0.01 to 1% in an aqueous solvent containing a buffer selected from a sodium phosphate buffer, a boric acid buffer and a sodium borate buffer.

## Patentansprüche

1. Wässrige, antiallergische, ophthalmische Lösung, die das Kaliumsalz des 9-Methyl-3-(1H-tetrazo1-5-yl)-4H-pyrido[1,2-a]pyrimidin-4-on in einer Konzentration von 0.01 bis 1% und einen Puffer, ausgewählt aus Natriumphosphat, Borsäure und Natriumborat, enthält.

2. Wässrige, antiallergische, ophthalmische Lösung gemäß Anspruch 1, in welcher der Puffer Dinatriumhydrogenphosphat und Natriumdihydrogenphosphat enthält.

3. Wässrige, antiallergische, ophthalmische Lösung gemäß Anspruch 2, in welcher die Konzentrationen des Dinatriumhydrogenphosphates und des Natriumdihydrogenphosphates 0.1 bis 2% beziehungsweise 0.002 bis 1% betragen.

4. Wässrige, antiallergische, ophthalmische Lösung gemäß Anspruch 1, in welcher der Puffer Dinatriumhydrogenphosphat und Kaliumdihydrogenphosphat enthält.

5. Wässrige, antiallergische, ophthalmische Lösung gemäß Anspruch 4, in welcher die Konzentrationen des Dinatriumhydrogenphosphates und des Kaliumdihydrogenphosphates 0.1 bis 1% beziehungsweise 0.002 bis 0.7% betragen.

6. Wässrige, antiallergische, ophthalmische Lösung gemäß Anspruch 1, in welcher der Puffer Borsäure und Natriumborat enthält.

7. Wässrige, antiallergische, ophthalmische Lösung gemäß Anspruch 6, in welcher die Konzentrationen der Borsäure und des Natriumborates 0.3 bis 2% beziehungsweise 0.3 bis 1% betragen.

8. Wässrige, antiallergische, ophthalmische Lösung gemäß Anspruch 1, in welcher der Puffer Borsäure und Monoethanolamin enthält.

9. Wässrige, antiallergische, ophthalmische Lösung gemäß Anspruch 8, in welcher die Konzentrationen der Borsäure und des Monoethanolamins 0.3 bis 2% beziehungsweise 0.1 bis 1% betragen.

10. Verfahren zur Herstellung einer wässrigen, antiallergischen, ophthalmischen Lösung, welches das Auflösen des Kaliumsalzes des 9-Methyl-3-(1H-tetrazol-5-yl)-4H-pyrido[1,2-a]pyrimidin-4-on in einer Konzentration von 0.01 bis 1% in einem wässrigen Lösungsmittel beinhaltet, welches einen Puffer, ausgewählt aus Natriumphosphat, Borsäure und Natriumborat, enthält.

## Revendications

1. Solution aqueuse anti-allergique ophtalmique contenant du sel potassique de la 9-méthyl-3-(1H-tétrazol-5-yl)-4H-pyrido[1,2-a]pyrimidine-4-one à une concentration comprise entre 0.01 et 1 % et un tampon choisi parmi un tampon au phosphate de sodium, un tampon à l'acide borique et un tampon au borate de sodium.

2. Solution aqueuse anti-allergique ophtalmique telle que revendiquée dans la Revendication 1, dans laquelle le tampon comprend du phosphate disodique et du phosphate mono-sodique.

3. Solution aqueuse anti-allergique ophtalmique telle que revendiquée dans la Revendication 2, dans laquelle les concentrations en phosphate disodique et en phosphate mono-sodique sont respectivement comprises entre 0.1 et 2 % et entre 0.002 et 1 %.

4. Solution aqueuse anti-allergique ophtalmique telle que revendiquée dans la Revendication 1, dans laquelle le tampon comprend du phosphate disodique et du phosphate mono-potassique.

5. Solution aqueuse anti-allergique ophtalmique telle que revendiquée dans la Revendication 4, dans laquelle les concentrations en phosphate disodique et en phosphate mono-potassique sont respectivement de 0.1 à 1 % et de 0.002 et 0.7 %.

6. Solution aqueuse anti-allergique ophtalmique telle que revendiquée dans la Revendication 1, dans laquelle le tampon comprend de l'acide borique et du borate de sodium.

7. Solution aqueuse anti-allergique ophtalmique telle que revendiquée dans la Revendication 6, dans laquelle les concentrations en acide borique et en borate de sodium sont respectivement comprises entre 0.3 et 2 % et entre 0.3 et 1 %.

8. Solution aqueuse anti-allergique ophtalmique telle que revendiquée dans la Revendication 1, dans laquelle le tampon comprend de l'acide borique et de la monoéthanolamine.

9. Solution aqueuse anti-allergique ophtalmique telle que revendiquée dans la Revendication 8, dans laquelle les concentrations en acide borique et en monoéthanolamine sont respectivement comprises entre 0.3 et 2 % et entre 0.1 et 1 %.

10. Procédé de préparation d'une solution aqueuse anti-allergique ophtalmique comprenant la dissolution de sel potassique de la 9-méthyl-3-(1H-tétrazol-5-yl)-4H-pyrido[1,2-a]pyrimidine-4-one à une concentration comprise entre 0.1 et 1 % dans un solvant aqueux contenant un tampon choisi parmi un tampon au phosphate de sodium, un tampon à l'acide borique et un tampon au borate de sodium.
